Europäisches Patentamt

European Patent Office

Office européen des brevets

(11) Publication number: 0 354 408 A1

# (12) EUROPEAN PATENT APPLICATION

(21) Application number: 89113735.8

(22) Date of filing: 25.07.89

(51) Int. Cl.⁴: G01N 33/577 , C12Q 1/48 , C12P 21/08

Claims for the following Contracting States: ES + GR.

(30) Priority: 12.08.88 IT 2170288

(43) Date of publication of application:
14.02.90 Bulletin 90/07

(84) Designated Contracting States:
AT BE CH DE ES FR GB GR IT LI LU NL SE

(71) Applicant: GRUPPO LEPETIT S.P.A.
23, Via G. Murat
I-20159 Milano(IT)

(72) Inventor: Nolli, Marialuisa
36, Via Filippo Corridoni
I-21053 Castellanza (VA)(IT)
Inventor: Soffientini, Adolfo
168, Via Fratelli di Dio
I-20099 Sesto San Giovanni (MI)(IT)

(74) Representative: Macchetta, Francesco et al
Gruppo Lepetit S.p.A. Patent and Trademark
Department 34, Via Roberto Lepetit
I-21040 Gerenzano (Varese)(IT)

(54) Anti-urokinase monoclonal antibodies.

(57) The present invention relates to a class of new IgG₁ monoclonal antibodies directed to an epitope on human urokinase which corresponds to the so-called GFD-region (growth factor domain-like region), i.e. an aminoacidic sequence similar to the one which was found on the epidermal growth factor. The main feature of the monoclonal antibodies of the invention is in fact that of selectively binding to said epitope without binding to any other epitope of urokinase and in particular without interfering with the binding of the enzyme to its substrate and thus without interfering with the specific enzymatic activity of urokinase using the F₆ fragment of urokinase.

EP 0 354 408 A1

# ANTI-UROKINASE MONOCLONAL ANTIBODIES

The present invention relates to a class of new IgG$_1$ monoclonal antibodies directed to an epitope on human urokinase which corresponds to the so-called GFD-region (growth factor domain-like region), i.e. an aminoacidic sequence similar to the one which was found on the epidermal growth factor. The main feature of the monoclonal antibodies of the invention is in fact that of selectively binding to said epitope without binding to any other epitope of urokinase and in particular without interfering with the binding of the enzyme to its substrate and thus without interfering with the specific enzymatic activity of urokinase.

The present invention encompasses also a method for preparing the monoclonal antibodies of the invention as well as analytical assays and purification procedures which employ them.

Human urokinase is a known plasminogen activator (PA) which is used in human therapy in particular in the treatment of myocardial infarct, thrombosis and embolism states. It is a serine protease of about 54,000, consisting of a light chain (A-chain, apparent molecular weight 20,000) and a heavy chain (B-chain; apparent molecular weight 30,000) which is oftentimes identified with the acronyms UK or tcuPA.

The natural precursor of urokinase, which is named prourokinase, differs structurally from it in that it is a single chain protein, which is oftentimes represented by the acronym "scuPA". Prourokinase (scuPA) is transformed into urokinase (tcuPA) by plasmin. ScuPA is believed to have a high therapeutic potential because of its possibly reduced side-effects.

Generally, urokinase is produced from biological sources, e.g. human urine, but also genetic engineering methods for producing it are described (e.g. EP-A-92182).

The isolation and characterization of a proteolytic fragment of urokinase was described by Stoppelli M.P. et al in Proc. Natl. Acad. Sci. USA 1985, 82, 4939-4943. Said fragment has been named ATF (amino terminal fragment).

It spans from aminoacid 1 to aminoacid 135 of human urokinase, is not required for the plasminogen activation and has a high affinity for the receptors of the surface of many cells such as the endotelial ones.

Several classes of monoclonal antibodies have been described since the pioneer work of C. Milstein and G. Köhler described in Nature, 256, 495-497 (1975).

Anti-urokinase monoclonal antibodies which may bind urokinase or prourokinase with different selectivity are for instance described in Belgian Patent 896253, Bioscience Report 3, 373-379 (1983), Thromb. Haemostas. 49, 24-27 (1983), EP-A-84344, Proc.Natl.Acad.Sci. USA, 81, 110-114 (1984) and 79, 3720-3723 (1982), Biochemistry 21, 6410-6415 (1982) and EP-A-122969.

None of the above publications describes however a monoclonal antibody capable of selectively binding to the urokinase or prourokinase region having a sequence similar to that of the epidermal growth factor (GFD-region) or a method for preparing or isolating any such monoclonal antibody.

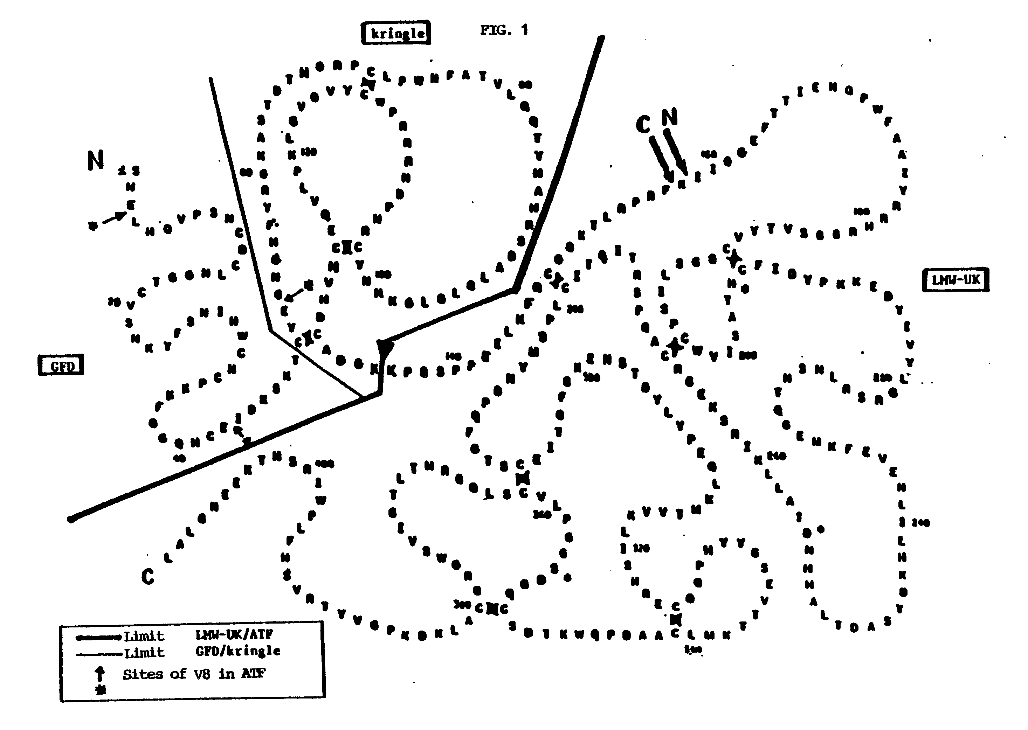

Fig. 1 represents an outline of the aminoacid sequnce of urokinase where the epitope which is bound by the monoclonal antibodies of the invention is highlighted.

The monoclonal antibodies of the invention are immunoglobulin of G class, preferably G$_1$, that are capable of selectively binding the urokinase or prourokinase region having a sequence which is similar to that of the epidermal growth factor, without binding to any epitope close to it, such as for instance the "kringle" (see Fig. 1) and without interfering with the enzymatic activity of urokinase or the enzymatic activation of prourokinase. Moreover, the monoclonal antibodies of the invention do not cross- react with any other urine or blood endopeptidases.

In view of their properties, the monoclonal antibodies of the invention can be used in the purification of urokinase, prourokinase or any fragment or derivative thereof that possesses the GFD-region.

Moreover, they can be used in assay systems that detect urokinase or prourokinase derivatives having the GFD-region altered, so that they are no longer bound by the monoclonal antibodies of the invention. In this case, by running a parallel assay with a monoclonal antibody that binds the antigen in a site other than the GFD, it is possible to identify the mutants having the GFD- region altered.

Another possible use of the monoclonal antibodies of this invention is in a conjuctive therapy with urokinase or prourokinase. In fact, given the affinity of these monoclonal antibodies for the cell-surface receptors of urokinase, including the endotelial ones, it is possible that their conjuctive administration with urokinase and/or pro-urokinase results in a reduction of side-effects due to urokinase-mediated activation of plasminogen in sites other than the vicinity of the blood cloths as well as in the reduction of the dosage of urokinase and/or pro-urokinase required for the therapeutic effect.

Other uses of the antibodies of the invention are easily found by the man skilled in the art on the basis of the present disclosure and the common knowledge in this art and are considered as encompassed by the scope of the present description and claims.

For conciseness, the monoclonal antibodies of the invention will be referred to also as "anti-GFD antibodies", thus referring to their most relevant feature, i.e., the property of selectively binding to an epitope of the GFD-region of urokinase or prourokinase.

The monoclonal antibodies of the invention are produced by the somatic cell hybridization technique of C. Milstein and G. Köhler described in Nature, 1975, 256, 495-497.

Briefly, spleen cells from animals immunized with the antigen are "fused", generally in the presence of a fusion promoter, with stabilized cell lines, such as tumor cell lines, e.g. myeloma cell lines. The used animals generally are small mammalians, preferably rodens, and in particular mice or rats.

There are many stabilized cell lines that are known and available to the public that can be conveniently used in the process of the invention.

Some of these cell lines are also deposited in internationally recognized repositories such as the American Type Culture Collection, (ATCC), Rockville, Mariland (USA). An example of said deposited cultures is the "non-secreting" murine myeloma PS/NS 1/1-Ag4-1, having accession No. ATCC TIB 18. The cell fusion is generally conducted in the presence of promoting agents such as Sendai virus or polyethylenglycols (e.g. PEG 1500 or 6000). The challanging antigen is generally highly purified in order to maximize the probability of obtaining spleen cells producing the desired antibodies and is selected from urokinase, prourokinase, ATF or a mixture thereof. The selection of the hybridomas which secern the monoclonal antibodies of the invention is preferably made according to a two-stage procedure.

In a first step, the monoclonal antibodies which bind to urokinase or prourokinase or ATF are selected, conveniently by an ELISA method, by using an affinity matrix bearing urokinase and/or prourokinase or ATF.

Then, the positive clones are assayed, generally with an immunoblotting assay, in the presence of a peptidic sequence corresponding to (being a relevant portion of or including) the urokinase GFD-region. An example of these peptidic sequences is represented by a urokinase fragment named $F_6$ having apparent molecular weight of about 6000 and corresponding to the human urokinase sequence spanning aproximately from Leucine 4 to Glutamic acid 43, and thus including, or being a relevant portion of, the GFD-region. Other sequences of this type that can be usefully employed can be obtained by genetic engineering as known in the art, on the basis of the knowledge of the above mentioned peptidic sequence, and include mutants and partial deletion or insertion products thereof. All these sequences will also be referred to as "GFD-like sequences". These sequences show the same immunological properties of the above mentioned urokinase sequence, i.e. are capable of selectively binding antibodies directed to the epitope on the urokinase GFD-region.

The hybridomas whose supernatant reacts with a GFD-like sequence (as defined above) and urokinase and/or prourokinase are further cloned and mass cultivated to obtain the monoclonal antibodies of the invention that are then isolated in pure form by the usual chromatographic techniques.

Fragment $F_6$ (like fragments $B_{11}$ or $B_{12}$ ) of urokinase or prourokinase is obtained by controlled treatment of urokinase or prourokinase or ATF (in the case of $B_{11}$ or $F_{12}$) in the presence of an enzyme capable of cutting a protein sequence close to a residue of glutamic acid, such as Staphylococcus aureus derived protease V8, which is commercially available. Under proper reaction conditions, in fact,this enzyme cuts the peptidic bond of an aminoacid sequence close to the carboxylic terminus of a residue of glutamic acid.

The following Examples further illustrate the invention.

Example 1 : Preparation of the anti-GFD monoclonal antibodies of the invention (DC 1, CD 6)

a) production of anti-urokinase monoclonal antibodies.

Highly purified 54,000 Dalton urokinase 120,000 IU/mg (PERSOLV [R] Lepetit) (or essentially pure prourokinase or ATF) is used to immunize 7-week old female Balb/C mice. 10 Microgram of·this urokinase in complete Freunds's adjuvant is injected into the peritoneum of the animals 60 days before fusion.

Another 10 microgram of urokinase is given to mice 30 days later. Then days before fusion, anti-urokinase antibody titre is measured by conventional ELISA method in blood samples taken from the tail veins and 5 days before fusion, a final booster of 10 microgram of urokinase is given endovenously only to those animals having an antibody titer higher than 1:10,000. Spleens are then removed on the day of the fusion and spleen cells (about $1 \times 10^8$) are fused with about $5 \times 10^7$ mouse myeloma NSO cells (a subline of P3/NS1/1Ag 4.1, ATCC accession number TIB 18) in 50% PEG 6000.

After fusion, which has been performed substantially following the method of C. Milstein and G. Köhler, the cells are resuspended in Dulbecco's Modified Eagle Medium (DMEM) supplemented with 10% fetal calf serum (FCS) and HAT medium (0.1mM Hypoxantine, 0.25 mM Aminopterine, 0.017 mM Thymidine, Flow laboratories) and put onto 5 different Costar plates containing mouse macrophages (feeder layer).

The HAT medium is changed every 3 days and, from day 10 after fusion, the samples of supernatants are collected and tested in the assay reported below (c.f. points b and c) to identify the epitope of urokinase to which they bind to.

The hybridomas which produce an antibody having the features of those of the invention (essentially : binding ability to the GFD-region of urokinase/prourokinase) are isolated, cloned under limit dilution conditions, mass cultivated and intraperitoneally injected into Balb/C mice previously treated with pristane (2,6, 10, 14-tetramethylpentyl decane; 0.5 ml). The ascitic fluids are collected after about 15 days from treatment. The concentration of monoclonal antibodies in these fluids is generally 5-20 mg/ml.

b) Screening for anti-urokinase monoclonal antibody-producing hybridomas by ELISA.

According to a modification of the Perlman method (Immunochemistry, 8_873 (1971)), flexible 96 well microtiter plates (Dynatech) are coated with 50 microliter/well of 20 microgram/ml solution of urokinase in phosphate buffered saline pH 7.2 (0.05 M sodium phosphate pH 7.2 which contains 0.5 M sodium chloride) and incubated for 1 h at room temperature. After a thorough washing with phosphate buffered saline containing 0.05% Tween 20, the wells are treated with 3% bovine serum albumine in phosphate buffered saline, for 3 h at room temperature to saturate possible sites of unspecific binding.

After washing carefully, 50 microliter of supernatant of hybridoma cultures (or the same volume of ascitic fluids) are added to the wells and left to react for two hours at room temperature. The plates are then washed and incubated with a 1:10000 dilution of rabbit-anti-mouse Ig conjugated with horseradish peroxidase (P161, DAKO), for 90 min at room temperature. After thorough washing, the plates are incubated with 200 microliter/well of a 1 mg/ml solution of the chromogen o-phenylethylendiamine (SIGMA) in 0.1 M citrate buffer pH 6, in the presence of 1.7 mM $H_2O_2$. The color is developed in 20 minutes and the optical density read at 492 nm against blanks without antigen or antibody. Wells with color levels four times higher than the blanks are considerd as positive.

c) immunoblotting assay for anti-GFD monoclonal antibodies and their epitopic characterization.

The clones which are positive in the above ELISA test (c.f. Example 1b) are further analyzed by an immunoblotting assay (Western blot) in the presence of scuPA, tcuPA, ATF and uPA fragments $F_{12}$, $B_{11}$ and $F_6$ (which are obtained as described in the Preparation reported below). These reference compounds are run in parallel in a SDS-PAGE (20% acrylamide; see below) and then blotted on a nitro-cellulose membrane that is treated with BSA (3% in phosphate buffer) for 1 h and exposed to the samples with the monoclonal antibodies (ascitic fluids, surnatant of cell cultures or any partially purified preparation thereof).

After rinsing, the antibodies which are specifically bound to the above fragments are revealed by horseraddish-labelled rabbit anti-mouse antibodies. By adding the chromogen (3-amino-9-ethyl carbazole) and incubating for 2 h at room temperature the color which developes is measured with a colorimeter.

In this experiment, the monoclonal antibodies of the invention bind tcuPA, scuPA, AFT and $F_6$ but do not bind $F_{12}$ or $B_{11}$. The hybridomas which secrete antibodies with these features are isolated and recloned as described in a) and their supernatants/ascitic liquors are purified as described below.

d) Purification of anti-urokinase monoclonal antibodies by affinity chromatography on Sepharose-urokinase.

A Sepharose-urokinase conjugate is prepared by reacting CNBr-activated Sepharose 4B (Pharmacia) with high molecular weight urokinase preferably in higly purified form (120,000 IU/mg). The coupling efficiency is of about 20 mg urokinase/ml swollen gel. Ascite fluids obtained as above are then applied to a Sepharose-urokinase affinity column (1.6 cm x 15 cm) pre-equilibrated in 0.05 M sodium phosphate buffer, containing 0.15 M NaCl pH 7.3. After rinsing with the same buffer, the selectively adsorbed anti-urokinase monoclonal antibodies are eluted with 0.1 M acetic acid pH 3.0. The antibody-containing fractions are pooled and concentrated by ultrafiltration on PSDE 09005 Millipore membranes and stored frozen until use. Two clones capable of producing substantial amounts of monoclonal antibodies with the features of those of the invention were identified and named DC1 and CD6.

e) alternatively, the mass cultivation of the hybridomas which produce the monoclonal antibodies of the invention can be according to a known cell cultivation system.

e.I) A 4 l bioreactor (Setric Genie Industrielle) equipped with means for the computerized control of the pH, the temperature and the dissolved oxigen is loaded with 2.5 l of DMEM medium containing 10% fetal calf serum and inoculated with a culture of the producing hybridoma at a concentration of about $10^5$ cell/ml. The cultivation is generally conducted for 10-15 days. After 5-6 days the maximum cell concentration is about $10^6$ cell/ml. Antibody production (about 70-100 microgram/ml) generally starts when the cell growth is at the plateau and increases up to 200-400 microgram/ml. Antibody production continues also when the cell concentration decreases up to about $10^5$ cell/ml.

e.II) A hollow fiber bioreactor (Vitafiber plus II, Amicon) with a fiber cut off of 30000 and equipped with computerized means to control the pH temperature, dissolved oxigen and flow of the culture medium is inoculated with a culture of the hybridoma producing the monoclonal antibody of the invention. $1-5 \times 10^8$ cells are inoculated in a 1000 fiber bioreactor (VF 2P30) in DMEM medium containing 10% FCS. The cells grow externally all around each fiber while the culture medium flows within the fiber. Because of the fiber cut off, the produced monoclonal antibody does not pass into the fiber lumen and thus it is not removed by the flowing medium but concentrates in the space between the fibers. Consequently, the antibody concentration is easily evaluated by sampling at intervals the cell supernatant and moreover it can easily be collected by removing portions of the supernatant when it reaches an optimal concentration. This bioreactor process can work 2-3 months producing 40-60 mg/day of antibody.

Example 2 : Evaluation of the purity and homogeneity of the monoclonal antibody preparations by gel-electrophoresis.

The monoclonal antibodies obtained above are submitted to SDS-PAGE (sodium dodecylphosphate polyacrylamide gel electrophoresis (see below) according to the method described by W.K. Laemmli in Nature 227, 680 (1970). Only the bands corresponding to the heavy and the light chains of IgG are evident indicating that the above eluate contains pure immunoglobulins.

Also the HPLC analysis confirms the presence of a single UV peak at 280 nm.

Preparation

Preparation of uPA fragments $F_6$, $B_{11}$ and $F_{12}$

a) preparation of fragments $F_6$, $B_{11}$ and $F_{12}$.

uPA amino-terminal fragment (ATF), (1.1 mg), is treated with Staphylococcus aureus derived protease V8 (0.32 mg; Boehringer) in sodium phosphate buffer (1.2 ml; 50 mM, pH 7.8) for 90 min at 37°C. Under these conditions the protease V8 cuts the ATF on the carboxylic side of glutamic acid residues. The enzymatic reaction is blocked by boiling the mixture 2 min and the proteic fragments so obtained are analyzed on SDS-PAGE (20% acrylamide) and compared to the original ATF. The band corresponding to ATF is no longer present, but three bands are observed with apparent molecular weight of 12000 ($F_{12}$) 11000 ($B_{11}$), and 6000 ($F_6$), respectively as determined by comparison with standards of given molecular weight.

b) separation of fragments $F_6$, $B_{11}$ and $F_{12}$

The above obtained mixture is loaded on a 5B4-agarose matrix (an agarose modified matrix bearing a monoclonal antibody, named 5B4, which binds the high molecular weight urokinase without binding the low molecular form which is prepared according to conventional methods by linking MAB 5B4 with Sepharose (Pharmacia) as described in EP-A-192675 and by Corti A. Nolli M.L., Soffientini A. and Cassani G. in Throm Haemostas 1986, 56, 219-224. This column was previously equilibrated with 0.15 M sodium chloride and 0.05 M sodium phosphate buffer pH 7.4 containing 1% polyethylenglycol (PEG) 6000.

c) recovery of the unbound fraction

A solution corresponding to the equilibrating buffer is passed through the column and fractions are collected, U.V. monitored (280 nm) and pooled depending on their content. Two major pooled fractions are obtained, one which contains the uPA fragment with apparent molecular weight of 12,000 (which is named $F_{12}$) and another one with apparent molecular weight of 6000 (which is named $F_6$). These fragments are purified by ion exchange chromatography followed by desalting in reverse phase chromatography as described below under e).

## d) recovery of the bound fraction

The fraction bound to the matrix is then eluted with 1 M sodium chloride and 0.1 M acetic acid containing 1% PEG 6000. Fractions are collected, monitored by U.V. (280 nm) and pooled according to their content. A single main fraction is obtained which contains the uPA fragment with apparent molecular weight of 11,000 which is named $B_{11}$ and is purified as described below.

## e) purification of fragments $B_{11}$, $F_6$ and $F_{12}$ .

The pooled fractions which contain, separately, fragment $B_{11}$, $F_{12}$ or $F_6$ obtained above, are further purified and concentrated by ion exchange chromatography in an FPLC (fast protein liquid chromatography) system (Pharmacia Fine Chemicals, Sweden) using a Mono S HR5/5 column (strong cation exchanger based on beaded hydrophilic resin with narrow particle size distribution; the charged group is $-CH_2SO_3$) pre-equilibrated with 0.05 M sodium acetate buffer, pH 4.8.

The elution is made with a linear gradient of aqueous sodium chloride from 0 to 1 M at 60 ml/h in 45 min. The fractions containing, separately, fragments $B_{11}$, $F_6$ and $F_{12}$ are then desalted; concentrated, and, if appropriate, further purified by reverse phase column chromatography with a FPLC (Fast Protein Liquid Chromatography) system (Pharmacia Fine Chemicals) using a pro-RPC HR5/10 reverse phase chromatographic column (macroporous, microparticulated silica with bonded $C_1/C_8$ groups), Eluting buffers: buffer A, 0.1% trifluoroacetic acid in water, and buffer B, 0.1% trifluoroacetic acid in acetonitrile; elution mode: 100% A for 20 min, linear gradient from to 60% B in A in 65 min, 100% B for 25 min; flow rate 0.3 ml/h.

The solvents of the pooled fractions separately containing fragments $F_6$, $F_{12}$ or $B_{11}$ are then evaporated under reduced pressure thus obtaining the single products in pure form.

## f) aminoacid sequencing of $F_6$, $B_{11}$ and $F_{12}$

The analysis of the N-terminal aminoacid sequence was made with an automatic sequencer (e.g. Applied Biosystem Model 470A, Gas-phase). Each of $F_{12}$ and $B_{11}$ resulted to be composed of a main fragment with minor contaminants, while fragment $F_6$ resulted to be a single peptide. This analysis shows also that fragment $B_{11}$ corresponds to the uPA portion known as "kringle" (which is bound by the previously known monoclonal antibody 5B4), and that fragment $F_{12}$ (which is not bound by MAB 5B4) differs from it only for a clipping between aminoacid 52 and 53 of the sequence of urokinase, while the molecular weight is substantially unchanged probably because of the presence of disulfide bonds that keep the parts together.

Fragment $F_6$ shows an apparent molecular weight substantially corresponding to that of the urokinase sequence spanning aproximately from Leucine 4 to Glutamic acid 43 and includes (or is included) in the GFD-region.

## SDS-PAGE

SDS-polyacrylamide gel electrophoresis (SDS-PAGE) is carried out as known in the art. One of the first description of this method was done by Laemmli UK, (Cleavage of structural proteins during the assembly of the head of bacteriophage T4, Nature 1970, 227:680-685).

The amount of acylamide in the employed gel may vary and is generally indicated (e.g. 20% acylamide, indicates the percentage of this compounds in the final gel). Generally, reference substances with known molecular weight are run in parallel with the test compounds.

## Claims

1. A monoclonal antibody specifically directed to the epitope of urokinase or prourokinase corresponding to the GFD-region, having the following features:
   a) it is of IgG class
   b) it is capable of selectively binding the GFD region of urokinase or prourokinase without binding to other urokinase immunogenic regions.

2. A monoclonal antibody specifically directed to the epitope of urokinase or prourokinase corresponding to the GFD region, having the following features:
   a) it is of IgG class
   b) it is capable of selectively binding the GFD-region of urokinase or prourokinase without binding to other urokinase regions
   c) it does not interfere with the enzymatic activity of urokinase or the enzymatic activation of prourokinase

3. A monoclonal antibody according to claim 1 or 2 which is of $IgG_1$ class.

4. A process for preparing an anti-GFD monoclonal antibody according to claims 1, 2 or 3 which comprises preparing a hybridoma capable of producing it by fusing spleen cells from experiment animals previously immunized with urokinase, prourokinase or ATF, or a mixture thereof, with a myeloma cell line, in the presence of a suitable fusion promoter and then isolating the desired producing hybridoma by an assay which identifies the anti-GFD monoclonal antibody by means of its specific binding to a growth factor domain-like aminoacid sequence.

5. A process according to claim 4 wherein the spleen cells and the myeloma cells are murine.

6. A process according to claim 4 wherein the fusion promoter is a polyethylenglycol.

7. A process according to claim 4 wherein the growth factor domain-like aminoacid sequence is a sequence corresponding to the amino acid sequence approximately spanning from Leucine 4 to Glutamic acid 43 of the sequence of urokinase or a mutation, deletion or insertion derivative thereof which maintains the same immunogenic properties.

8. A process for preparing a compound according to claims 1, 2 or 3 which comprises cultivating, under appropriate conditions, a hybridoma according to claim 4.

9. Use of a monoclonal antibody of claim 1, 2 or 3 in the preparation of an affinity matrix for the purification or determination of urokinase or prourokinase.

10. Process for identifying · or purifying urokinase or prourokinase derivatives having the GFD-region altered which comprises contacting a preparation of the test substance in parallel or sequentially with:

a) an anti-urokinase antibody directed to any epitope of the molecule other than the GFD-region,

b) an antibody of claims 1, 2 or 3, and then detecting or isolating those compounds that give a positive reaction with a) and a negative reaction with b).

CLAIM FOR THE CONTRACTING STATES: ES, GR.

1. A process for preparing a monoclonal antibody specifically directed to the epitope of urokinase or prourokinase corresponding to the GFD-region, having the following features:

a) it is of IgG class

b) it is capable of selectively binding the GFD-region of urokinase or prourokinase, without binding to other urokinase immunogenic regions, which comprises: preparing a hybridoma capable of producing it by fusing spleen cells from experiment animals previously immunized with urokinase, prourokinase or ATF, or a mixture thereof, with a myeloma cell line, in the presence of a suitable fusion promoter and then isolating the desired producing hybridoma by an assay which identifies the produced anti-GFD monoclonal antibody by means of its specific binding to a growth factor domain-like aminoacid sequence.

2. A process according to claim 1 for preparing an anti-GFD monoclonal antibody having the following features

a) it is of IgG class

b) it is capable of selectively binding the GFD-region of urokinase or prourokinase without binding to other urokinase regions

c) it does not interfere with the enzymatic activity of urokinase or the enzymatic activation of prourokinase

3. A process according to claim 1 or 2 wherein the obtained anti-GFD monoclonal antibody is of $IgG_1$ class.

4. A process according to claim 1, 2 or 3 wherein the spleen cells and the myeloma cells are murine.

5. A process according to claim 1, 2 or 3 wherein the fusion promoter is a polyethylenglycol.

6. A process according to claim 1, 2 or 3 wherein the growth factor domain-like aminoacid sequence is a sequence corresponding to the amino acid sequence approximately spanning from Leucine 4 to Glutamic acid 43 of the sequence of urokinase or a mutation, deletion or insertion derivative thereof which maintains the same immunogenic properties.

7. A process for preparing an anti-GFD monoclonal antibody according to claims 1, 2 or 3 which comprises cultivating, under appropriate conditions, a hybridoma having the features reported in claim 1.

8. Use of a monoclonal antibody of claim 1, 2 or 3 in the preparation of an affinity matrix for the purification or determination of urokinase or prourokinase.

9. Process for identifying or purifying urokinase or prourokinase derivatives having the GFD-region altered which comprises contacting a preparation of the test substance in parallel or sequentially with:

a) an anti-urokinase antibody directed to any epitope of the molecule other than the GFD-region,

b) an antibody of claims 1, 2 or 3, and then detecting or isolating those compounds that give a positive reaction with a) and a negative reaction with b).

FIG. 1

| DOCUMENTS CONSIDERED TO BE RELEVANT | | | EP 89113735.8 |
|---|---|---|---|

| Category | Citation of document with indication, where appropriate, of relevant passages | Relevant to claim | CLASSIFICATION OF THE APPLICATION (Int. Cl.4) |
|---|---|---|---|
| D,A | BE - A - 896 253 (REGION WALLONNE) * Claims * -- | 1,3,4, 9,10 | G 01 N 33/577 C 12 Q 1/48 C 12 P 21/08 |
| A | EP - A2 - 0 084 344 (ASAHI KASEI KOGYO KABUSHIKI KAISHA) * Claims 1-7,11,12 * -- | 1,2,7, 11,12, 15 | |
| A | CHEMICAL ABSTRACTS, vol. 96, no. 7, February 15, 1982, Columbus, Ohio, USA P.HERION et al. "Monoclonal antibodies against urokinase" page 450, Abstract-no. 50 340p & Biosci. Rep. 1981, 1(11), 885-92 -- | 1,3,4, 9 | |
| P,A | DD - A1 - 264 025 (AKADEMIE DER WISSENSCHAFTEN DER DDR) * Abstract; pages 1,2 * ---- | 1,4,5, 9 | TECHNICAL FIELDS SEARCHED (Int. Cl.4) G 01 N 33/00 C 12 P 21/00 C 12 Q C 12 N 5/00 C 12 N 15/00 |

The present search report has been drawn up for all claims

| Place of search | Date of completion of the search | Examiner |
|---|---|---|
| VIENNA | 09-11-1989 | SCHNASS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503 03 82